# EUROPEAN PATENT APPLICATION

(11) **EP 4 450 619 A1**
(43) Date of publication of application: **23.10.2024**
(21) Application number: 22907471.1
(22) Date of filing: 14.12.2022
(51) Int. Cl.: C12N 9/12, C12N 1/15, C12N 1/19, C12N 1/21, C12N 5/10, C12N 15/54, C12N 15/63, C12P 19/34

(54) **REVERSE TRANSCRIPTASE HAVING EXCELLENT THERMAL STABILITY**

(30) Priority: 17.12.2021 JP 2021205525
(71) Applicant: TOYOBO CO., LTD., Osaka-shi, Osaka 530-0001 (JP); Shizuoka Prefectural University Corporation, Shizuoka-shi, Shizuoka 422-8526 (JP)
(72) Inventor: YOKOE, Sho, Tsuruga-shi, Fukui 914-8550 (JP); OCHI, Kensuke, Tsuruga-shi, Fukui 914-8550 (JP); NAKANO, Shogo, Shizuoka-shi, Shizuoka 422-8526 (JP); ITO, Sohei, Shizuoka-shi, Shizuoka 422-8526 (JP)
(74) Representative: Vossius & Partner Patentanwälte Rechtsanwälte mbB
(86) International application number: PCT/JP2022/045987
(87) International publication number: WO 2023/112947

(57) **Abstract**

Provided is a novel reverse transcriptase with excellent thermal stability. A reverse transcriptase comprising the following amino acid sequence: (a) an amino acid sequence having at least 75% identity with the amino acid sequence represented by SEQ ID NO: 2 or 3; or (b) an amino acid sequence having deletion, substitution, insertion, or addition of one or several amino acids in the amino acid sequence represented by SEQ ID NO: 2 or 3.

## Description

### Technical Field

The present invention relates to a reverse transcriptase. More specifically, the present invention relates to a reverse transcriptase with excellent thermal stability, a reverse transcription method using the reverse transcriptase, a polynucleotide encoding the reverse transcriptase, a kit comprising the reverse transcriptase, and the like.

### Background Art

Reverse transcriptases generally have an activity of synthesizing cDNA using RNA as a template (hereinafter referred to as "RNA-dependent DNA polymerase activity") and an activity of degrading RNA strands of RNA/DNA hybrids (hereinafter referred to as "RNase H activity"). Reverse transcriptases are used, for example, for the analysis of the base sequence of mRNA that directly reflects the amino acid sequence of protein expressed in the living body, the construction of cDNA libraries, RT-PCR, and the like. Moloney murine leukemia virus reverse transcriptase (MMLV), avian myeloblastosis virus reverse transcriptase (AMV), etc., are conventionally known as reverse transcriptases used for such purposes.

If mRNA has a base sequence that tends to form secondary structures, the secondary structures interfere with cDNA synthesis by reverse transcriptases. Accordingly, it is desirable to synthesize cDNA while suppressing the formation of secondary structures by raising the reaction temperature. However, the Moloney murine leukemia virus reverse transcriptase and avian myeloblastosis virus reverse transcriptase often have low thermal stability, and may be inactivated at high temperatures at which the formation of RNA secondary structures is suppressed. Therefore, in recent years, various reverse transcriptases having higher thermal stability than wild-type reverse transcriptase and having improved reactivity even at 42 to 60°C, at which stability is generally low, have been developed, for example, by introducing multiple amino acid mutations into reverse transcriptases (PTL 1, PTL 2, and NPL 1). However, reverse transcriptases with further improved stability are still desired.

The common conventional method for improving reverse transcriptases has been to introduce mutations in one or several amino acids, or at most ten or more amino acids, and perform evaluation. However, this method requires a huge number of experiments to select combinations of mutations. Therefore, in practice, researchers often narrow down mutations to some extent based on their experience and intuition, and select and evaluate them. For this reason, many of the reverse transcriptase variants that have been discovered so far have relatively high homology to the amino acid sequence of wild-type reverse transcriptase. In addition, mutations often target amino acids in a helix or sheet structure in contact with the template, and even when mutating loop structures, most of the mutations target amino acid residues in a region in contact with the template.

### Citation List

### Patent Literature

PTL 1: JP2000-139457A
PTL 2: JP6180002B

### Non-patent Literature

NPL 1: Journal of Biotechnology, Vol. 150, Issue 3, pp. 299-306 (published in 2010)

### Summary of Invention

### Technical Problem

An object of the present invention is to provide a novel reverse transcriptase with improved thermal stability.

### Solution to Problem

As a result of intensive studies in view of the above problem, the present inventors found a reverse transcriptase having reverse transcriptase activity (hereinafter also referred to as "reverse transcription activity") and improved thermal stability, thereby arriving at the present invention.

### [Item 1]

A reverse transcriptase comprising the following amino acid sequence:
(a) an amino acid sequence having at least 75% identity with the amino acid sequence represented by SEQ ID NO: 2 or 3; or
(b) an amino acid sequence having deletion, substitution, insertion, or addition of one or several amino acids in the amino acid sequence represented by SEQ ID NO: 2 or 3.

### [Item 2]

The reverse transcriptase according to Item 1, which has the following amino acid residues (i) to (vi) in the amino acid sequence (a) or (b):
(i) an amino acid at a position corresponding to position 67 is L or M,
(ii) an amino acid at a position corresponding to position 175 is D,
(iii) an amino acid at a position corresponding to position 229 is L or I,
(iv) an amino acid at a position corresponding to position 308 is A,
(v) an amino acid at a position corresponding to position 437 is A or T, and
(vi) an amino acid at a position corresponding to position 592 is A.

### [Item 3]

The reverse transcriptase according to Item 1 or 2, which has at least one amino acid residue selected from the group consisting of the following (1) to (43) in the amino acid sequence (a) or (b):
(1) an amino acid at a position corresponding to position 5 is L,
(2) an amino acid at a position corresponding to position 47 is P,
(3) an amino acid at a position corresponding to position 51 is V,
(4) an amino acid at a position corresponding to position 52 is E,
(5) an amino acid at a position corresponding to position 62 is V,
(6) an amino acid at a position corresponding to position 85 is L,
(7) an amino acid at a position corresponding to position 87 is V,
(8) an amino acid at a position corresponding to position 89 is R,
(9) an amino acid at a position corresponding to position 94 is A,
(10) an amino acid at a position corresponding to position 144 is R,
(11) an amino acid at a position corresponding to position 160 is P,
(12) an amino acid at a position corresponding to position 219 is T,
(13) an amino acid at a position corresponding to position 232 is D,
(14) an amino acid at a position corresponding to position 233 is T,
(15) an amino acid at a position corresponding to position 235 is E,
(16) an amino acid at a position corresponding to position 266 is E,
(17) an amino acid at a position corresponding to position 276 is G,
(18) an amino acid at a position corresponding to position 338 is T,
(19) an amino acid at a position corresponding to position 350 is K,
(20) an amino acid at a position corresponding to position 354 is S,
(21) an amino acid at a position corresponding to position 377 is I,
(22) an amino acid at a position corresponding to position 414 is I,
(23) an amino acid at a position corresponding to position 419 is L,
(24) an amino acid at a position corresponding to position 425 is D,
(25) an amino acid at a position corresponding to position 429 is L,
(26) an amino acid at a position corresponding to position 463 is S,
(27) an amino acid at a position corresponding to position 476 is P,
(28) an amino acid at a position corresponding to position 477 is A,
(29) an amino acid at a position corresponding to position 495 is D,
(30) an amino acid at a position corresponding to position 519 is E,
(31) an amino acid at a position corresponding to position 523 is F,
(32) an amino acid at a position corresponding to position 532 is D,
(33) an amino acid at a position corresponding to position 542 is V,
(34) an amino acid at a position corresponding to position 569 is K,
(35) an amino acid at a position corresponding to position 573 is L,
(36) an amino acid at a position corresponding to position 578 is R,
(37) an amino acid at a position corresponding to position 581 is I,
(38) an amino acid at a position corresponding to position 597 is A,
(39) an amino acid at a position corresponding to position 600 is Q,
(40) an amino acid at a position corresponding to position 632 is A,
(41) an amino acid at a position corresponding to position 646 is V,
(42) an amino acid at a position corresponding to position 658 is K, and
(43) an amino acid at a position corresponding to position 662 is L.

### [Item 4]

The reverse transcriptase according to any one of Items 1 to 3, which has at least one amino acid residue selected from the group consisting of the following (2), (4), (6), (8), (9), (11), (12), (13), (15), (16), (19), (21), (24), (26), (27), (29), (33), (34), (38), (39), and (41) in the amino acid sequence (a) or (b):
(2) an amino acid at a position corresponding to position 47 is P,
(4) an amino acid at a position corresponding to position 52 is E,
(6) an amino acid at a position corresponding to position 85 is L,
(8) an amino acid at a position corresponding to position 89 is R,
(9) an amino acid at a position corresponding to position 94 is A,
(11) an amino acid at a position corresponding to position 160 is P,
(12) an amino acid at a position corresponding to position 219 is T,
(13) an amino acid at a position corresponding to position 232 is D,
(15) an amino acid at a position corresponding to position 235 is E,
(16) an amino acid at a position corresponding to position 266 is E,
(19) an amino acid at a position corresponding to position 350 is K,
(21) an amino acid at a position corresponding to position 377 is I,
(24) an amino acid at a position corresponding to position 425 is D,
(26) an amino acid at a position corresponding to position 463 is S,
(27) an amino acid at a position corresponding to position 476 is P,
(29) an amino acid at a position corresponding to position 495 is D,
(33) an amino acid at a position corresponding to position 542 is V,
(34) an amino acid at a position corresponding to position 569 is K,
(38) an amino acid at a position corresponding to position 597 is A,
(39) an amino acid at a position corresponding to position 600 is Q, and
(41) an amino acid at a position corresponding to position 646 is V.

### [Item 5]

The reverse transcriptase according to any one of Items 1 to 4, which has at least one amino acid residue selected from the group consisting of the following (2), (5), (10), (14), (20), (25), (27), (29), (33), and (43) in the amino acid sequence (a) or (b):
(2) an amino acid at a position corresponding to position 47 is P,
(5) an amino acid at a position corresponding to position 62 is V,
(10) an amino acid at a position corresponding to position 144 is R,
(14) an amino acid at a position corresponding to position 233 is T,
(20) an amino acid at a position corresponding to position 354 is S,
(25) an amino acid at a position corresponding to position 429 is L,
(27) an amino acid at a position corresponding to position 476 is P,
(29) an amino acid at a position corresponding to position 495 is D,
(33) an amino acid at a position corresponding to position 542 is V, and
(43) an amino acid at a position corresponding to position 662 is L.

### [Item 6]

A reverse transcriptase having at least 80% identity with the amino acid sequence represented by SEQ ID NO: 1, and having at least one amino acid residue selected from the group consisting of the following (1) to (43):
(1) an amino acid at a position corresponding to position 5 is L,
(2) an amino acid at a position corresponding to position 47 is P,
(3) an amino acid at a position corresponding to position 51 is V,
(4) an amino acid at a position corresponding to position 52 is E,
(5) an amino acid at a position corresponding to position 62 is V,
(6) an amino acid at a position corresponding to position 85 is L,
(7) an amino acid at a position corresponding to position 87 is V,
(8) an amino acid at a position corresponding to position 89 is R,
(9) an amino acid at a position corresponding to position 94 is A,
(10) an amino acid at a position corresponding to position 144 is R,
(11) an amino acid at a position corresponding to position 160 is P,
(12) an amino acid at a position corresponding to position 219 is T,
(13) an amino acid at a position corresponding to position 232 is D,
(14) an amino acid at a position corresponding to position 233 is T,
(15) an amino acid at a position corresponding to position 235 is E,
(16) an amino acid at a position corresponding to position 266 is E,
(17) an amino acid at a position corresponding to position 276 is G,
(18) an amino acid at a position corresponding to position 338 is T,
(19) an amino acid at a position corresponding to position 350 is K,
(20) an amino acid at a position corresponding to position 354 is S,
(21) an amino acid at a position corresponding to position 377 is I,
(22) an amino acid at a position corresponding to position 414 is I,
(23) an amino acid at a position corresponding to position 419 is L,
(24) an amino acid at a position corresponding to position 425 is D,
(25) an amino acid at a position corresponding to position 429 is L,
(26) an amino acid at a position corresponding to position 463 is S,
(27) an amino acid at a position corresponding to position 476 is P,
(28) an amino acid at a position corresponding to position 477 is A,
(29) an amino acid at a position corresponding to position 495 is D,
(30) an amino acid at a position corresponding to position 519 is E,
(31) an amino acid at a position corresponding to position 523 is F,
(32) an amino acid at a position corresponding to position 532 is D,
(33) an amino acid at a position corresponding to position 542 is V,
(34) an amino acid at a position corresponding to position 569 is K,
(35) an amino acid at a position corresponding to position 573 is L,
(36) an amino acid at a position corresponding to position 578 is R,
(37) an amino acid at a position corresponding to position 581 is I,
(38) an amino acid at a position corresponding to position 597 is A,
(39) an amino acid at a position corresponding to position 600 is Q,
(40) an amino acid at a position corresponding to position 632 is A,
(41) an amino acid at a position corresponding to position 646 is V,
(42) an amino acid at a position corresponding to position 658 is K, and
(43) an amino acid at a position corresponding to position 662 is L.

### [Item 7]

The reverse transcriptase according to Item 6, which has at least 80% identity with the amino acid sequence represented by SEQ ID NO: 1, and has at least one amino acid residue selected from the group consisting of the following (2), (4), (6), (8), (9), (11), (12), (13), (15), (16), (19), (21), (24), (26), (27), (29), (33), (34), (38), (39), and (41):
(2) an amino acid at a position corresponding to position 47 is P,
(4) an amino acid at a position corresponding to position 52 is E,
(6) an amino acid at a position corresponding to position 85 is L,
(8) an amino acid at a position corresponding to position 89 is R,
(9) an amino acid at a position corresponding to position 94 is A,
(11) an amino acid at a position corresponding to position 160 is P,
(12) an amino acid at a position corresponding to position 219 is T,
(13) an amino acid at a position corresponding to position 232 is D,
(15) an amino acid at a position corresponding to position 235 is E,
(16) an amino acid at a position corresponding to position 266 is E,
(19) an amino acid at a position corresponding to position 350 is K,
(21) an amino acid at a position corresponding to position 377 is I,
(24) an amino acid at a position corresponding to position 425 is D,
(26) an amino acid at a position corresponding to position 463 is S,
(27) an amino acid at a position corresponding to position 476 is P,
(29) an amino acid at a position corresponding to position 495 is D,
(33) an amino acid at a position corresponding to position 542 is V,
(34) an amino acid at a position corresponding to position 569 is K,
(38) an amino acid at a position corresponding to position 597 is A,
(39) an amino acid at a position corresponding to position 600 is Q, and
(41) an amino acid at a position corresponding to position 646 is V.

### [Item 8]

The reverse transcriptase according to Item 6 or 7, which has at least 80% identity with the amino acid sequence represented by SEQ ID NO: 1, and has at least one amino acid residue selected from the group consisting of the following (2), (5), (10), (14), (20), (25), (27), (29), (33), and (43):
(2) an amino acid at a position corresponding to position 47 is P,
(5) an amino acid at a position corresponding to position 62 is V,
(10) an amino acid at a position corresponding to position 144 is R,
(14) an amino acid at a position corresponding to position 233 is T,
(20) an amino acid at a position corresponding to position 354 is S,
(25) an amino acid at a position corresponding to position 429 is L,
(27) an amino acid at a position corresponding to position 476 is P,
(29) an amino acid at a position corresponding to position 495 is D,
(33) an amino acid at a position corresponding to position 542 is V, and
(43) an amino acid at a position corresponding to position 662 is L.

### [Item 9]

The reverse transcriptase according to any one of Items 1 to 8, wherein an amino acid sequence of one or more regions selected from the group consisting of (A) a region corresponding to positions 91 to 105, (B) a region corresponding to positions 109 to 120, (C) a region corresponding to positions 125 to 138, (D) a region corresponding to positions 146 to 157, (E) a region corresponding to positions 182 to 205, (F) a region corresponding to positions 220 to 228, (G) a region corresponding to positions 251 to 263, (H) a region corresponding to positions 302 to 319, (I) a region corresponding to positions 351 to 358, and (J) a region corresponding to positions 391 to 404 of the amino acid sequence represented by SEQ ID NO: 1, 2, or 3 has at least 90% identity with an amino acid sequence of a corresponding region in the amino acid sequence of SEQ ID NO: 1, 2, or 3.

### [Item 10]

The reverse transcriptase according to any one of Items 1 to 9, wherein the amino acid sequence (a) is an amino acid sequence having at least 85% identity with the amino acid sequence represented by SEQ ID NO: 2 or 3.

### [Item 11]

The reverse transcriptase according to any one of Items 1 to 10, which lacks RNase H activity.

### [Item 12]

The reverse transcriptase according to any one of Items 1 to 11, which has a residual activity of 70% or more when heated at 50°C for 10 minutes.

### [Item 13]

The reverse transcriptase according to any one of Items 1 to 12, which has a residual activity of 20% or more when heated at 55°C for 10 minutes.

### [Item 14]

A polynucleotide encoding the reverse transcriptase according to any one of Items 1 to 13.

### [Item 15]

A vector comprising the polynucleotide according to Item 14.

### [Item 16]

A cell transformed with the vector according to Item 15.

### [Item 17]

A reagent comprising the reverse transcriptase according to any one of Items 1 to 13, the polynucleotide according to Item 14, the vector according to Item 15, and/or the cell according to Item 16.

### [Item 18]

A method for producing the reverse transcriptase according to any one of Items 1 to 13 using the polynucleotide according to Item 14, the vector according to Item 15, the cell according to Item 16, and/or the reagent according to Item 17.

### [Item 19]

A method for synthesizing cDNA from an RNA template using the reverse transcriptase according to any one of Items 1 to 13.

### [Item 20]

The method according to Item 19, which is a RT-PCR method further comprising performing a PCR reaction.

### [Item 21]

A kit comprising the reverse transcriptase according to any one of Items 1 to 13.

### [Item 22]

The kit according to Item 21, for use in synthesis of cDNA using RNA as a template.

### Advantageous Effects of Invention

The present invention provides a novel and useful reverse transcriptase with improved thermal stability. The reverse transcriptase with improved thermal stability can synthesize more cDNA than wild-type reverse transcriptase even when reverse transcription reactions are performed from RNAs with higher-order structures, and can synthesize cDNA from various templates.

### Brief Description of Drawings

Fig. 1 shows the results of electrophoresis in Example 5.
Fig. 2 shows the results of aligning the amino acid sequences of variant reverse transcriptases G2 and G6 with the amino acid sequence of wild-type reverse transcriptase (MMLV).
Fig. 3 shows the results of electrophoresis in Example 7.
Fig. 4 shows highly conserved regions in the alignment of the amino acid sequences of variant reverse transcriptases G2, G6, and G7 with the amino acid sequence of wild-type reverse transcriptase.
Fig. 5 shows the results of evaluating cDNA synthesis ability in Example 9.
Fig. 6 shows the results of performing a one-step qRT-PCR reaction using a G6-derived variant reverse transcriptase in Example 10.
Fig. 7 shows the results of performing a one-step qRT-PCR reaction using a G2-derived variant reverse transcriptase in Example 11.

### Description of Embodiments

The present invention is described in detail below; however, the present invention is not limited thereto.

The present invention provides a novel reverse transcriptase with improved thermal stability.

In the present specification, the "reverse transcriptase" refers to an enzyme having an activity of synthesizing cDNA using RNA as a template (hereinafter also referred to as "RNA-dependent DNA polymerase activity," "reverse transcription activity," "reverse transcriptase activity," etc.), and may or may not have RNase H activity. The presence or absence of RNA-dependent DNA polymerase activity can be confirmed by the method for measuring reverse transcription activity described later. The reverse transcriptases may include wild-type reverse transcriptase and variant reverse transcriptases obtained by artificially introducing mutations into the amino acid sequence of the wild-type reverse transcriptase.

In the present specification, "wild-type reverse transcriptase" (hereinafter also referred to as "WT") refers to a reverse transcriptase into which no mutation is artificially introduced. Examples of the wild-type reverse transcriptase include a reverse transcriptase comprising the amino acid sequence represented by SEQ ID NO: 1. The "amino acid sequence represented by SEQ ID NO: 1" refers to the amino acid sequence represented by SEQ ID NO: 1 shown in the sequence listing (the amino acid sequence of Moloney murine leukemia virus reverse transcriptase). Further, "Moloney murine leukemia virus reverse transcriptase" is also expressed as "MMLV reverse transcriptase."

The novel reverse transcriptase of the present invention has an amino acid sequence different from that of conventionally known wild-type reverse transcriptase. Therefore, in the present specification, the reverse transcriptase of the present invention is also referred to as "variant reverse transcriptase" or "modified reverse transcriptase." In the present specification, the terms "variant" and "modified" in "variant reverse transcriptase" and "modified reverse transcriptase" are used interchangeably, and mean that they have an amino acid sequence different from conventionally known reverse transcriptases; these terms are not used to distinguish between artificial mutations and natural mutations. Therefore, the variant reverse transcriptase of the present invention refers to a reverse transcriptase having an amino acid sequence different from SEQ ID NO: 1, which represents the sequence of the wild-type reverse transcriptase, obtained by modifying one or more amino acids in the amino acid sequence represented by SEQ ID NO: 1. It does not matter whether the variant reverse transcriptase is a variant reverse transcriptase obtained by an artificial mutation or a variant reverse transcriptase resulting from a naturally occurring mutation.

In the present specification, simplified symbols in alphabetical notation may be used for base sequences, amino acid sequences, and their individual constituents; however, all follow the practice in the fields of molecular biology and gene engineering. Further, in the present specification, expressions such as "I5L" are used to briefly indicate a mutation in the amino acid sequence. "I5L" indicates substitution of isoleucine (I) at the 5th position with leucine (L). That is, it shows the type and location of amino acid residue before substitution, and the type of amino acid residue after substitution. Further, SEQ ID NOs correspond to SEQ ID NOs shown in the sequence listing unless otherwise specified. In the case of multiple mutations, the above expressions are expressed by connection with "/" (e.g., A47P/P52E/Q85L/V89R/P94A/R160P/I219T/S232D/L235E/Q266E). In the present specification, in an amino acid sequence that is not completely identical to the amino acid sequence represented by SEQ ID NO: 2 or 3, the position corresponding to a certain position (Xth position) on SEQ ID NO: 2 or 3 refers to the position corresponding to this position of SEQ ID NO: 2 or 3 when the primary structures of the sequences are compared (aligned).

In one embodiment, the variant reverse transcriptase preferably has low amino acid sequence identity with conventional wild-type reverse transcriptase (MMLV reverse transcriptase). Such a variant reverse transcriptase has reverse transcription activity and improved thermal stability although the amino acid sequence identity with the conventional wild-type reverse transcriptase is relatively low.

In one embodiment, the variant reverse transcriptase has the following amino acid sequences (a) and/or (b):
(a) an amino acid sequence having at least 75% identity with the amino acid sequence represented by SEQ ID NO: 2 or 3; or
(b) an amino acid sequence having substitution, deletion, insertion, or addition of one or several amino acids in the amino acid sequence represented by SEQ ID NO: 2 or 3.

In one embodiment, the variant reverse transcriptase has the above amino acid sequence, and has reverse transcriptase activity and thermal stability (e.g., a residual activity of 70% or more when heated at 50°C for 10 minutes, or a residual activity of 20% or more when heated at 55°C for 10 minutes) .

The reverse transcriptase having the amino acid sequence represented by SEQ ID NO: 2 or 3 has an amino acid sequence identity of 69.9% or 69.7%, respectively, with the wild-type reverse transcriptase (MMLV). Reverse transcriptases having such a low amino acid sequence identity with MMLV have not been known so far. The amino acid sequence identity between SEQ ID NO: 2 and SEQ ID NO: 3 is 67.0%. Thus, since similar reverse transcriptase activity and thermal stability are exhibited even when the amino acid sequence identity is low, it can be understood that the reverse transcriptase of the present invention is not limited to those comprising the amino acid sequence represented by SEQ ID NO: 2 or 3, and may be those obtained by further modifying these amino acid sequences.

In one embodiment, the variant reverse transcriptase can have modification at a certain rate in the amino acid sequence of SEQ ID NO: 2 or 3. In one embodiment, the variant reverse transcriptase preferably has at least 80% identity with the amino acid sequence represented by SEQ ID NO: 1. The variant reverse transcriptase is not particularly limited as long as the reverse transcription activity and/or thermal stability is not lost. For example, the amino acid sequence preferably has at least 75%, at least 80%, at least 85%, at least 90%, at least 95%, at least 96%, at least 97%, at least 98%, or at least 99%, identity with the amino acid sequence represented by SEQ ID NO: 1, 2, or 3. The amino acid sequence identity can be evaluated by any method known in the art. For example, the amino acid sequence identity can be calculated using a commercially available analysis tool or an analysis tool available through a telecommunication line (Internet). For example, the amino acid sequence identity can be calculated using the default parameters of the National Center for Biotechnology Information (NCBI) homology algorithm BLAST (Basic Local Alignment Search Tool; http://www.ncbi.nlm.nih.gov/BLAST/). Further, the amino acid sequence of the modified reverse transcriptase may be an amino acid sequence having deletion, substitution, insertion, and/or addition of one or several amino acids in the amino acid sequence represented by SEQ ID NO: 2 or 3. The number of amino acids indicated by "one or several" is not particularly limited as long as the reverse transcription activity and/or thermal stability is not lost. For example, "one or several" refers to 1 to 30, further 1 to 20, preferably 1 to 10, more preferably 1 to 5, and even more preferably 1 to 3. The amino acid sequence as described above may be, for example, artificially produced by a genetic engineering method, or may be an amino acid sequence of a naturally occurring protein.

In one embodiment, when the variant reverse transcriptase has substitution in the amino acid sequence of SEQ ID NO: 1, 2, or 3, the substitution is preferably substitution between structurally and/or chemically similar amino acids (so-called conservative substitution). Examples of conservative substitutions include, but are not limited to, substitution between basic amino acids (H, K, and R), substitution between acidic amino acids (D and E), substitution between neutral nonpolar amino acids (A, V, L, I, P, F, M, and W), substitution between neutral polar amino acids (G, N, Q, S, T, V, and C), substitution between aromatic amino acids (W, F, H, and Y), substitution between nitrogen-containing amino acids (K, R, N, Q, and P), substitution between sulfur-containing amino acids (C and M), substitution between oxygen-containing amino acids (S and T), substitution between β-branched amino acids (V, L, and I), and substitution between amino acids having a linear alkyl or hydrogen side chain (A and G).

In one embodiment, the variant reverse transcriptase preferably has amino acid residues specified in the Examples provided later as amino acid residues that characterize the reverse transcriptase. Specific examples of such amino acid residues include the following amino acid residues (i) to (vi). The reverse transcriptase of the present invention can have any one of the following amino acid residues (i) to (vi), but preferably has 2 or more, 3 or more, 4 or more, or 5 or more of (i) to (vi), and particularly preferably all of the 6 amino acid residues.

### Examples of Amino Acid Residue Characterizing Reverse Transcriptase

(i) an amino acid at a position corresponding to position 67 is L or M,
(ii) an amino acid at a position corresponding to position 175 is D,
(iii) an amino acid at a position corresponding to position 229 is L or I,
(iv) an amino acid at a position corresponding to position 308 is A,
(v) an amino acid at a position corresponding to position 437 is A or T, and
(vi) an amino acid at a position corresponding to position 592 is A.

In a specific preferred embodiment, the variant reverse transcriptase may have, as the above amino acid residues that characterize the reverse transcriptase, 6 amino acid residues in the order of 67th, 175th, 229th, 308th, 437th, and 592nd that are any of LDLAAA, MDIAAA, MDIATA, MDLAAA, or MDLATA. Preferred among these is one having amino acid residues LDLAAA, MDIATA, or MDLAAA, and particularly preferred is one having amino acid residues LDLAAA or MDIATA. Due to the possession of such 6 amino acid residues, the enzyme can have reverse transcription activity more reliably.

In one embodiment, the variant reverse transcriptase preferably has amino acid residues characteristic to the amino acid sequences of the reverse transcriptases represented by SEQ ID NOs: 2 and 3. Examples of such amino acid residues include amino acid residues commonly found in the amino acid sequences represented by SEQ ID NO: 2 and SEQ ID NO: 3, unlike the amino acid sequence of wild-type reverse transcriptase (MMLV). Due to the possession of such amino acid residues characteristic to the amino acid sequences of SEQ ID NOs: 2 and 3, superior thermal stability, which is different from wild-type reverse transcriptase and which the reverse transcriptases represented by SEQ ID NOs: 2 and 3 of the present invention commonly have, can be exhibited more reliably. Examples of such amino acid residues include the following amino acid residues (1) to (43).

### Amino Acid Residues Characteristic to Transcriptases Represented by SEQ ID NOs: 2 and 3

(1) an amino acid at a position corresponding to position 5 is L,
(2) an amino acid at a position corresponding to position 47 is P,
(3) an amino acid at a position corresponding to position 51 is V,
(4) an amino acid at a position corresponding to position 52 is E,
(5) an amino acid at a position corresponding to position 62 is V,
(6) an amino acid at a position corresponding to position 85 is L,
(7) an amino acid at a position corresponding to position 87 is V,
(8) an amino acid at a position corresponding to position 89 is R,
(9) an amino acid at a position corresponding to position 94 is A,
(10) an amino acid at a position corresponding to position 144 is R,
(11) an amino acid at a position corresponding to position 160 is P,
(12) an amino acid at a position corresponding to position 219 is T,
(13) an amino acid at a position corresponding to position 232 is D,
(14) an amino acid at a position corresponding to position 233 is T,
(15) an amino acid at a position corresponding to position 235 is E,
(16) an amino acid at a position corresponding to position 266 is E,
(17) an amino acid at a position corresponding to position 276 is G,
(18) an amino acid at a position corresponding to position 338 is T,
(19) an amino acid at a position corresponding to position 350 is K,
(20) an amino acid at a position corresponding to position 354 is S,
(21) an amino acid at a position corresponding to position 377 is I,
(22) an amino acid at a position corresponding to position 414 is I,
(23) an amino acid at a position corresponding to position 419 is L,
(24) an amino acid at a position corresponding to position 425 is D,
(25) an amino acid at a position corresponding to position 429 is L,
(26) an amino acid at a position corresponding to position 463 is S,
(27) an amino acid at a position corresponding to position 476 is P,
(28) an amino acid at a position corresponding to position 477 is A,
(29) an amino acid at a position corresponding to position 495 is D,
(30) an amino acid at a position corresponding to position 519 is E,
(31) an amino acid at a position corresponding to position 523 is F,
(32) an amino acid at a position corresponding to position 532 is D,
(33) an amino acid at a position corresponding to position 542 is V,
(34) an amino acid at a position corresponding to position 569 is K,
(35) an amino acid at a position corresponding to position 573 is L,
(36) an amino acid at a position corresponding to position 578 is R,
(37) an amino acid at a position corresponding to position 581 is I,
(38) an amino acid at a position corresponding to position 597 is A,
(39) an amino acid at a position corresponding to position 600 is Q,
(40) an amino acid at a position corresponding to position 632 is A,
(41) an amino acid at a position corresponding to position 646 is V,
(42) an amino acid at a position corresponding to position 658 is K, and
(43) an amino acid at a position corresponding to position 662 is L.

The variant reverse transcriptase preferably has any one of the above amino acid residues (1) to (43), more preferably 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, 27 or more, 28 or more, 29 or more, 30 or more, 31 or more, 32 or more, 33 or more, 34 or more, 35 or more, 36 or more, 37 or more, 38 or more, 39 or more, 40 or more, 41 or more, or 42 or more of (1) to (43), and particularly preferably all of the above 43 amino acid residues.

Reverse transcriptases (MMLV) generally have a three-dimensional structure in which two-dimensional structures of protein, such as α-helices, β-sheets, and loops, are intricately intertwined, and are known to form 5 domains (fingers, palm, thumb, connection, and RNase H domains). The amino acid residues (1) to (43) characteristic to the reverse transcriptases represented by SEQ ID NOs: 2 and 3 are unevenly distributed in the loop regions, and particularly are often found in the fingers domain, the palm domain, or loop regions in the vicinity of these domains. The fingers domain is known to sandwich the template and incorporate the substrate. The palm domain is known to contain activity centers (positions D224 and D225) inside. Although not wishing to be bound by any theory, it is assumed that the reverse transcriptase having the amino acid sequence of SEQ ID NO: 2 or 3 changes its conformation, flexibility, chemical properties, etc. in the fingers domain, the palm domain, or loop regions in the vicinity of these domains, thereby increasing its structural stability, while maintaining or improving its binding to the template RNA, to achieve both reverse transcription activity and high thermal stability. From this point of view, in a specific embodiment, the reverse transcriptase of the present invention preferably has, as the amino acid residues characteristic to the reverse transcriptases represented by SEQ ID NOs: 2 and 3, amino acid residues in the fingers domain, the palm domain, or loop regions in the vicinity of these domains, and preferably has, for example, amino acid residues (1) to (17) among the above amino acid residues characteristic to the reverse transcriptases of SEQ ID NOs: 2 and 3.

In one embodiment, the variant reverse transcriptase preferably has high conservation of the following regions (A) to (J) of the amino acid sequence of SEQ ID NO: 1, 2, or 3.
(A): positions 91 to 105 (Thumb top)
(B): positions 109 to 120 (Thumb top 2)
(C): positions 125 to 138 (Thumb-Palm)
(D): positions 146 to 157 (Palm activity center)
(E): positions 182 to 205 (Thumb middle)
(F): positions 220 to 228 (Palm activity center side)
(G): positions 251 to 263 (Palm activity center side)
(H): positions 302 to 319 (Finger)
(I): positions 351 to 358 (Finger)
(J): positions 391 to 404 (Finger-RNase H)

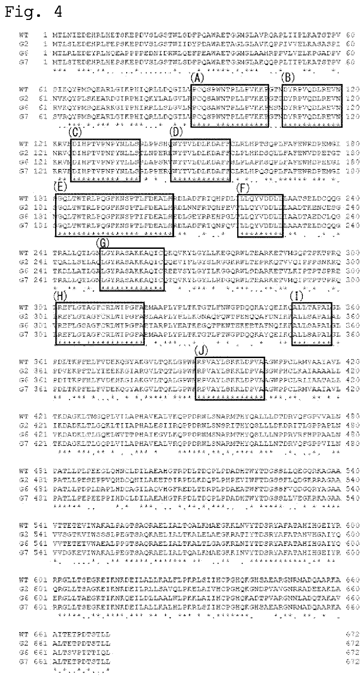

These regions have common amino acid sequences with the wild-type reverse transcriptase (SEQ ID NO: 1) and the variant reverse transcriptases (SEQ ID NOs: 2 to 4) (Fig. 4). In one embodiment, the variant reverse transcriptase has at least a certain level of identity with the amino acid sequence of SEQ ID NO: 1, 2, or 3, and the amino acid sequence of a region corresponding to one or more regions selected from the group consisting of (A) to (J) preferably has at least 90%, at least 95%, at least 96%, at least 98%, at least 99%, or 100%, identity with the amino acid sequence of SEQ ID NO: 1, 2, or 3. The number of one or more regions is preferably 2 or more, 3 or more, 4 or more, 5 or more, 6 or more, 7 or more, 8 or more, 9 or more, or 10.

In a more specific embodiment, the variant reverse transcriptase preferably has, among the amino acid residues (1) to (43) characteristic to the amino acid sequences of the reverse transcriptases represented by SEQ ID NOs: 2 and 3, those that have significantly different structural and/or chemical properties from amino acid residues at corresponding positions in the wild-type reverse transcriptase. It is assumed that amino acid residues that have significantly different properties from the amino acid residues in the wild-type reverse transcriptase greatly contribute to the high thermal stability of the reverse transcriptase of the present invention, which has not been observed in the wild-type reverse transcriptase. Such an amino acid residue can be any of the following (2), (4), (6), (8), (9), (11), (12), (13), (15), (16), (19), (21), (24), (26), (27), (29), (33), (34), (38), (39), or (41).

Amino Acid Residues Characteristic to Reverse Transcriptases Represented by SEQ ID NOs: 2 and 3 and Having Different Properties from Wild-Type
(2) an amino acid at a position corresponding to position 47 is P,
(4) an amino acid at a position corresponding to position 52 is E,
(6) an amino acid at a position corresponding to position 85 is L,
(8) an amino acid at a position corresponding to position 89 is R,
(9) an amino acid at a position corresponding to position 94 is A,
(11) an amino acid at a position corresponding to position 160 is P,
(12) an amino acid at a position corresponding to position 219 is T,
(13) an amino acid at a position corresponding to position 232 is D,
(15) an amino acid at a position corresponding to position 235 is E,
(16) an amino acid at a position corresponding to position 266 is E,
(19) an amino acid at a position corresponding to position 350 is K,
(21) an amino acid at a position corresponding to position 377 is I,
(24) an amino acid at a position corresponding to position 425 is D,
(26) an amino acid at a position corresponding to position 463 is S,
(27) an amino acid at a position corresponding to position 476 is P,
(29) an amino acid at a position corresponding to position 495 is D,
(33) an amino acid at a position corresponding to position 542 is V,
(34) an amino acid at a position corresponding to position 569 is K,
(38) an amino acid at a position corresponding to position 597 is A,
(39) an amino acid at a position corresponding to position 600 is Q, and
(41) an amino acid at a position corresponding to position 646 is V.

In a preferred embodiment, the variant reverse transcriptase may have 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, or 20 or more, out of the 21 amino acid residues: (2), (4), (6), (8), (9), (11), (12), (13), (15), (16), (19), (21), (24), (26), (27), (29), (33), (34), (38), (39), and (41), and particularly preferably all of the 21 amino acid residues, in an amino acid sequence having at least a certain level of identity with the amino acid sequence of SEQ ID NO: 1, 2, or 3. Of these amino acid residues, it is preferable to have one or more amino acid residues selected from (2), (4), (6), (8), (9), (11), (12), (13), (15), and (16), which reside in the fingers domain, the palm domain, or loop regions in the vicinity of these domains.

Further, as shown in the Examples provided later, it has been confirmed that a reverse transcriptase that has been found to have thermal stability similar to that of the reverse transcriptase of the present invention (reverse transcriptase having the amino acid sequence represented by SEQ ID NO: 4) and the amino acid sequences represented by SEQ ID NOs: 2 and 3 share a characteristic amino acid residue that is not found in the wild-type MMLV reverse transcriptase. Therefore, in a specific embodiment, the reverse transcriptase of the present invention preferably has an amino acid residue common in SEQ ID NOs: 2, 3, and 4. Such an amino acid residue can be any of the following (2), (5), (10), (14), (20), (25), (27), (29), (33), or (43).

### Amino Acid Residues Characteristic to Reverse Transcriptases Represented by SEQ ID NOs: 2, 3, and 4

(2) an amino acid at a position corresponding to position 47 is P,
(5) an amino acid at a position corresponding to position 62 is V,
(10) an amino acid at a position corresponding to position 144 is R,
(14) an amino acid at a position corresponding to position 233 is T,
(20) an amino acid at a position corresponding to position 354 is S,
(25) an amino acid at a position corresponding to position 429 is L,
(27) an amino acid at a position corresponding to position 476 is P,
(29) an amino acid at a position corresponding to position 495 is D,
(33) an amino acid at a position corresponding to position 542 is V, and
(43) an amino acid at a position corresponding to position 662 is L.

In a preferred embodiment, the reverse transcriptase of the present invention may have 2 or more, 3 or more, 4 or more, 5 or more, 6 or more, 7 or more, 8 or more, or 9 or more, out of the 10 amino acid residues: (2), (5), (10), (14), (20), (25), (27), (29), (33), and (43), and particularly preferably all of the 10 amino acid residues, in an amino acid sequence having at least a certain level of identity with the amino acid sequence of SEQ ID NO: 1, 2, or 3. Of these amino acid residues, it is preferable to have one or more amino acid residues selected from (2), (5), (10), and (14), which reside in the fingers domain, the palm domain, or loop regions in the vicinity of these domains.

The reverse transcriptase of the present invention can be composed of an amino acid sequence containing an amino acid residue as described above. A person skilled in the art could have produced a reverse transcriptase protein with a target amino acid sequence using any genetic engineering method known in the art, for example, by appropriately designing a base sequence encoding the target amino acid sequence, incorporating it into an expression vector etc., and transforming it into host cells for expression.

In a specific embodiment, the variant reverse transcriptase of the present invention may be one that lacks RNase activity. Examples of reverse transcriptases lacking RNase activity include, but are not limited to, those in which aspartic acid at position 524 (corresponding to position 525 of SEQ ID NOs: 1 to 3) is substituted with alanine and/or those in which aspartic acid at position 583 (corresponding to position 584 of SEQ ID NOs: 1 to 3) is substituted with asparagine. The RNase activity of wild-type reverse transcriptase may degrade RNA, which is a template for the reverse transcription reaction. In particular, this activity can be a problem in the synthesis of cDNA using long-chain RNA (e.g., full-length RNA) as a template. Reverse transcriptases modified to lack RNase activity are preferable because in a reverse transcription reaction using a long-chain RNA as a template, it is possible to suppress the degradation of the RNA strand template during the reaction.

The reverse transcriptase of the present invention is characterized by having reverse transcription activity and high thermal stability in combination. The reverse transcription activity and thermal stability can be specifically confirmed by the following measurement methods. For variant reverse transcriptases that are in a predetermined relationship with the amino acid sequence represented by SEQ ID NO: 1, 2, or 3, a person skilled in the art would have been able to evaluate the presence or absence of reverse transcription activity and thermal stability by the method described below, and obtain such variant reverse transcriptases.

### Method for Measuring Reverse Transcription Activity

In the present specification, the reverse transcription activity of reverse transcriptases can be measured by the following procedure. In this measurement method, when the enzyme activity is high, a sample containing the measurement target may be suitably diluted for the measurement.

First, 10 µL of A liquid, 22 µL of B liquid, and 1 µL of C liquid, all of which are previously prepared as described below, and 12 µL of sterilized water are added to a reaction vessel, such as a microtube, and the mixture is stirred and mixed. Then, 5 µL of a sample solution containing the measurement target or a diluted solution thereof is added and allowed to react at 42°C for 10 minutes. Thereafter, the resultant is cooled, and 150 µL of the following D liquid is added and stirred, and then ice-cooled for 10 minutes. The resulting liquid is filtered through a glass filter (Whatman GF/C filter), and sufficiently washed with 0.1 N hydrochloric acid and 100% ethanol. The radioactivity of the filter is measured using a liquid scintillation counter (Tri-Carb 2810 TR, produced by Packard), and the uptake of nucleotides is measured. One unit of enzyme activity is the amount of enzyme that incorporates 1 nmole of nucleotide into the acid-insoluble fraction per 10 minutes under these conditions.

### Reverse Transcriptase Activity Measurement Reagent

- A liquid: 250 mM Tris-HCl (pH: 8.3), 375 mM potassium chloride, 15 mM magnesium chloride, 50 mM dithiothreitol
- B liquid: 1 mg/mL poly A, 1 pmol/µL dT20, 10 mM dTTP
- C liquid: [3H]-dTTP
- D liquid: 0.07 M sodium pyrophosphate, 0.7 M trichloroacetic acid

### Measurement of Thermal Stability (Residual Activity of Reverse Transcription Activity after Heat Treatment)

The variant reverse transcriptases to be measured are each diluted with a storage buffer (50 mM Tris-HCl (pH: 7.5), 300 mM KCl, 50% glycerol, 0.1 mM EDTA) to 100 U/µL, and the reverse transcription activity value before storage is measured according to the procedure described in the section "Method for Measuring Reverse Transcription Activity" above. Then, the variant reverse transcriptases to be measured diluted with the above storage buffer are stored under specific storage conditions (e.g., storage conditions in an incubator at 45°C to 55°C for 5 to 15 minutes; in a preferred embodiment, storage conditions in an incubator at 50°C for 10 minutes or storage conditions in an incubator at 55°C for 10 minutes) . After a predetermined time has passed from the start of storage (e.g., after 5 to 15 minutes; in a preferred embodiment, after 10 minutes), the reverse transcription activity value after storage is measured according to the procedure described in the section "Method for Measuring Reverse Transcription Activity" above, as in the case before storage. Then, the residual activity can be calculated by dividing the reverse transcription activity value after storage by the reverse transcription activity value before storage, as shown in the following formula I. Residual activity (%) = (reverse transcription activity value after storage/ reverse transcription activity value before storage) × 100

The reverse transcriptase of the present invention preferably exhibits higher thermal stability than wild-type reverse transcriptase. In a preferred embodiment, the reverse transcriptase of the present invention can be a reverse transcriptase having a residual activity of 50% or more, further 60% or more, preferably 70% or more, more preferably 75% or more, and particularly preferably 80% or more, for example, when heated at 50°C for 10 minutes. In a specific embodiment, the reverse transcriptase of the present invention can be a reverse transcriptase having a residual activity of 90% or more when heated at 50°C for 10 minutes.

In a further embodiment, the reverse transcriptase of the present invention can be a reverse transcriptase that is stable even when heated at a higher temperature (55°C). For example, the reverse transcriptase of the present invention can be a reverse transcriptase having a residual activity of 20% or more, preferably 25% or more, and more preferably 29% or more, when heated at 55°C for 10 minutes. In a specific embodiment, the reverse transcriptase of the present invention can be a reverse transcriptase having a residual activity of 40% or more when heated at 55°C for 10 minutes.

From yet another perspective, the reverse transcriptase of the present invention can be a variant reverse transcriptase having higher residual activity than that of wild-type reverse transcriptase, for example, when heated at 50°C and/or 55°C over 10 minutes. A specific example can be a reverse transcriptase having a residual activity about 1.5 times or more, and preferably about 2.0 times or more, higher than that of wild-type reverse transcriptase when heated at 50°C and/or 55°C for 10 minutes. In a specific preferred embodiment, the reverse transcriptase of the present invention can be a reverse transcriptase having a residual activity about 2.5 times or more, and more preferably 2.8 times or more, higher than that of wild-type reverse transcriptase, for example, when heated at 50°C and/or 55°C for 10 minutes.

In a further embodiment, the present invention provides a polynucleotide encoding the reverse transcriptase of the present invention described above. The polynucleotide encoding the reverse transcriptase refers to, for example, a polynucleotide from which the protein of the reverse transcriptase of the present invention can be obtained when the polynucleotide is expressed by a conventional method. That is, this polynucleotide refers to a polynucleotide comprising a base sequence corresponding to the amino acid sequence of the protein of the reverse transcriptase of the present invention. A person skilled in the art could have easily determined a base sequence corresponding to a predetermined amino acid sequence according to a codon table etc. known in the art. Further, the polynucleotide encoding the reverse transcriptase of the present invention also includes a polynucleotide that differs due to codon degeneracy. The polynucleotide can be any nucleic acid polymer, such as DNA or RNA.

In a further embodiment, the present invention provides a vector comprising the polynucleotide. Specifically, the polynucleotide encoding the reverse transcriptase is transferred to a vector (e.g., an expression vector or a cloning vector), if necessary. Any vector may be used as long as it allows cloning and/or expression etc. of the reverse transcriptase of the present invention. For example, a plasmid can be used. Examples of plasmids include, but are not limited to, pUC118, pUC18, pBR322, pBluescript, pLED-M1, p73, pGW7, pET3a, pET8c, pET23b, and the like.

In a further embodiment, the present invention provides a cell transformed with the vector. Such a cell can be preferably used to express the protein encoding the reverse transcriptase of the present invention. In a specific preferred embodiment, the recombinant host cell of the present invention is obtained by transforming a host cell using the above expression vector. Examples of the host cell include *Escherichia coli* and yeast; *Escherichia coli* is particularly preferred. Examples of *Escherichia coli* include *Escherichia coli* DH5α, JM109, HB101, XL1Blue, PR1, HS641(DE3), BL21(DE3), and the like. That is, it is preferable in the present invention to insert the gene encoding the reverse transcriptase into the above vector to obtain an expression vector, and transform the host cell with the expression vector.

In one embodiment, the expression vector of the present invention may contain elements for facilitating the purification of the reverse transcriptase, such as extracellular signals and His tags.

A further embodiment provides a method for producing the reverse transcriptase using the polynucleotide, the vector, the transformed cell, and/or a reagent containing one or more of them. For example, the host cell is transformed using the expression vector, and then applied to an agar medium containing a drug such as ampicillin to form colonies. The colonies are inoculated into a nutrient medium, such as LB medium or 2 × YT medium, and cultured at 37°C for 12 to 20 hours. Then, the cells are disrupted to extract a crude enzyme solution. Any known method may be used to disrupt the cells. For example, sonication, physical disruption such as French press or glass bead disruption, or lytic enzymes such as lysozyme can be used. Any method may be used to obtain the purified reverse transcriptase from the obtained crude enzyme solution. The reverse transcriptase of the present invention can be isolated, for example, by subjecting the crude enzyme solution to centrifugation, ultracentrifugation, ultrafiltration, salting-out, dialysis, ion-exchange column chromatography, adsorption column chromatography, affinity chromatography, gel filtration column chromatography, or the like.

The present invention further provides a reverse transcription method characteristically using the reverse transcriptase of the present invention. The reverse transcription method of the present invention is characterized by synthesizing cDNA from an RNA template using the variant reverse transcriptase of the present invention. The reverse transcription method of the present invention may also be a RT-PCR method further combining a PCR reaction step (e.g., a qRT-PCR method such as a one-step qRT-PCR method or a two-step qRT-PCR method). The reverse transcriptase of the present invention has higher thermal stability than wild-type reverse transcriptase. Accordingly, the reverse transcription method of the present invention allows reverse transcription reactions in a wide temperature range (e.g., up to 50°C or up to around 55°C), including temperatures high enough to suppress the formation of RNA secondary structures. Therefore, the reverse transcription method of the present invention is highly versatile because reverse transcription reactions using, for example, RNA that tends to form secondary structures as a template, can be performed efficiently regardless of the type of RNA.

In one preferred embodiment, in the reverse transcription method of the present invention, the reverse transcription reaction can be performed by incubating the reverse transcriptase, RNA as a template, an oligonucleotide primer complementary to a part of the RNA, and four types of deoxyribonucleoside triphosphates in a reverse transcription reaction buffer.

The reaction temperature in the reverse transcription reaction differs depending on the type of RNA used, the type of reverse transcriptase used, etc., and is preferably suitably set according to the type of RNA used, the type of reverse transcriptase used, etc. The reaction temperature can be set to 37 to 42°C, for example, when the RNA used does not tend to form secondary structures. Further, for example, when the RNA used tends to form secondary structures, the reaction temperature can be set to a temperature higher than the reaction temperature suitable for wild-type reverse transcriptase, for example, 42 to 55°C. Since the reverse transcriptase of the present invention has high thermal stability, there is the advantage that the reverse transcription reaction can be sufficiently performed even under conditions with high reaction temperatures. The reaction time is, for example, about 1 minute to 1 hour, and preferably about 3 minutes to 30 minutes, and more preferably about 5 minutes to 10 minutes, but is not limited thereto.

The reverse transcription reaction buffer used in the reverse transcription method of the present invention may contain divalent cations, such as magnesium ions and manganese ions. The concentration of divalent cations is preferably suitably set according to the type of reverse transcriptase and other components contained in the reverse transcription reaction buffer. For example, the divalent cation concentration of the reverse transcription reaction buffer is set to 1 to 30 mM. Further, the reverse transcription reaction buffer may contain, if necessary, a reducing agent (e.g., dithiothreitol), a stabilizer (e.g., glycerol or trehalose), an organic solvent (e.g., dimethyl sulfoxide or formamide), and other components as long as they do not impair the object of the present invention.

In a further embodiment, the present invention provides a reagent containing the reverse transcriptase, the polynucleotide, the vector, and/or the cell. In the case of a reagent used for RT-PCR methods, it is preferable to further contain components for performing PCR reactions (e.g., DNA polymerases, primers, and optionally probes). Such a reagent may contain other optional components (e.g., stabilizers, preservatives, and other optional additives) depending on the purpose of use etc. For example, the reagent containing the reverse transcriptase may further contain the reverse transcription reaction buffer and the like. The reagent of the present invention is not particularly limited in its use, and can be suitably used, for example, for the reverse transcription reaction that synthesizes cDNA using RNA as a template. Further, the reagent of the present invention can also be preferably used for producing the reverse transcriptase of the present invention.

In a further embodiment, the present invention provides a kit comprising the reverse transcriptase, the polynucleotide, the vector, the cell, and/or a reagent containing one or more of them. The kit of the present invention can be, for example, a kit for performing the reverse transcription reaction of synthesizing cDNA using RNA as a template, a kit for producing the reverse transcriptase of the present invention, or the like, but is preferably a kit for synthesizing cDNA using RNA as a template (this kit is also referred to as "reverse transcription reaction kit" etc.).

In one embodiment, the reverse transcription reaction kit of the present invention is a kit for performing a reverse transcription reaction, and one of its features is that the kit contains the reverse transcriptase of the present invention (including a case in which it is provided as a reagent containing the reverse transcriptase). Since the reverse transcription reaction kit of the present invention contains the reverse transcriptase of the present invention, which has high thermal stability, it can be preferably used even in reverse transcription reactions in a wide temperature range, including temperatures high enough to suppress the formation of RNA secondary structures. In addition, since thermal stability is improved, it is possible to detect a low-concentration template. Thus, this kit is highly convenient. The kit of the present invention may further contain, for example, an instruction manual for performing a reverse transcription reaction using the reverse transcriptase of the present invention. The kit of the present invention can be provided in the form in which the reverse transcriptase etc. are packed, for example, in a single package, and in which information on how to use the kit is included.

In a specific embodiment, for example, in the reverse transcription reaction kit, reagents necessary for performing the reverse transcription reaction may be enclosed in containers different from the container containing the reverse transcriptase. If the progress of the reverse transcription reaction during storage of the reagents is stopped, the reagents may be enclosed in the same container as the reverse transcriptase. The reagents may be enclosed in a container in amounts suitable for performing the reverse transcription reaction. This eliminates the need to mix the reagents in amounts suitable for the reverse transcription reaction, which facilitates handling.

### Examples

The present invention is described in more detail below with reference to Examples. The present invention is not limited to the Examples.

### Example 1: Screening of Reverse Transcriptase Gene by Bioinformatics

In this Example, an attempt was made to genetically design enzymes using bioinformatics technology from the big data of a huge number of known protein groups. Specifically, using sequence alignment of protein amino acid sequences, a library containing multiple proteins with amino acid sequences similar to known protein amino acid sequences with the target enzyme activity was created from the existing database, and novel reverse transcriptases were screened by identifying at least two correlated residues among them, and selecting and evaluating proteins with the correlated residues.

In this Example, using the amino acid sequence of the wild-type reverse transcriptase represented by SEQ ID NO: 1 as an indicator, 5000 similar sequences were selected by BLASTp. Among them, extremely long or short sequences were removed by curation to narrow down the number of sequences to 450 sequences. The selected amino acid sequences were then analyzed using the statistical method described in JP2018-88864A, which is incorporated herein by reference in its entirety. Classification was performed from the obtained results, and six amino acid residues (positions 67, 175, 229, 308, 437, and 529 of SEQ ID NO: 1) that can classify reverse transcriptases were identified as LDLAAA, MDIAAA, MDIATA, MDLAAA, and MDLATA. Then, only the similar sequences with these six amino acid residues were selected, and in those sequences, amino acids relatively conserved as the amino acid sequences of the wild-type reverse transcriptase were partially substituted to obtain multiple candidate amino acid sequences.

### Example 2: Production of Protein Expression Vector of Reverse Transcriptase Gene

Two of the amino acid sequences obtained in Example 1 were selected, and protein expression vectors were produced. Specifically, two DNA sequences (SEQ ID NOs: 5 and 6) were obtained from the two (G2 and G6) candidate amino acid sequences based on the codon usage of Escherichia coli. These were each cloned into pET-23b(+) to produce plasmids (pG2 and pG6) into which the candidate reverse transcriptase sequence was incorporated. The obtained plasmids were transformed into BL21-Gold competent cells (Agilent Technologies) and used for enzyme preparation.

### Example 3: Acquisition of Reverse Transcriptase

The cells obtained in Example 2 were cultured as described below. First, 80 mL of sterilized TB medium (Molecular Cloning, 2nd Edition, p.A. 2) containing 100 ug/mL ampicillin was dispensed into a 500-mL Sakaguchi flask. In this medium, a plasmid transformed strain previously cultured at 37°C for 16 hours in 3 mL of LB medium (1% bactotrypton, 0.5% yeast extract, 0.5% sodium chloride) containing 100 ug/mL ampicillin was inoculated and aerobically cultured at 30°C for 16 hours. Then, IPTG (produced by Nacalai Tesque, Inc.) was added to a final concentration of 0.1 mM, and the cells were aerobically cultured at 30°C for another 4 hours. The cells were collected from the culture medium by centrifugation and suspended in 50 mL of disruption buffer (10 mM Tris-HCl (pH: 7.5), 300 mM KCl, 5% glycerol). Then, the cells were disrupted by sonication to obtain a cell disruption solution. Next, the cell disruption solution was purified with His GraviTrap (produced by GE Healthcare). The washing conditions were 10 mM Tris-HCl (pH: 7.5), 300 mM KCl, 5% glycerol, and 50 mM imidazole. The elution conditions were 10 mM Tris-HCl (pH: 7.5), 300 mM KCl, 5% glycerol, and 300 mM imidazole. Finally, substitution was carried out using a storage buffer (50 mM Tris-HCl (pH: 7.5), 300 mM KCl, 50% glycerol, 0.1 mM EDTA), thereby obtaining each reverse transcriptase.

The activity of the reverse transcriptases purified as described above was measured in the following manner. When the enzyme activity was high, the sample was diluted for the measurement.

### Reverse Transcriptase Activity Measurement Reagent

A liquid: 250 mM Tris-HCl (pH: 8.3), 375 mM potassium chloride, 15 mM magnesium chloride, 50 mM dithiothreitol
B liquid: 1 mg/mL poly A, 1 pmol/µL dT20, 10 mM dTTP
C liquid: [3H]-dTTP
D liquid: 0.07 M sodium pyrophosphate, 0.7 M trichloroacetic acid

### Method for Measuring Reverse Transcriptase Activity

10 µL of A liquid, 22 µL of B liquid, 1 µL of C liquid, and 12 µL of sterilized water were added to a microtube, and the mixture was stirred and mixed. Then, 5 µL of the above purified enzyme diluted solution was added, and allowed to react at 42°C for 10 minutes. Thereafter, the resultant was cooled, and 150 µL of D liquid was added and stirred, and then ice-cooled for 10 minutes. This liquid was filtered through a glass filter (Whatman GF/C filter), and sufficiently washed with 0.1 N hydrochloric acid and ethanol. The radioactivity of the filter was measured using a liquid scintillation counter (Tri-Carb 2810 TR, produced by Packard), and the uptake of nucleotides was measured. One unit of enzyme activity was the amount of enzyme that incorporated 1 nmole of nucleotide into the acid-insoluble fraction per 10 minutes under this condition.

As a result of the above measurement, it was confirmed that the two candidate reverse transcriptases (G2 and G6) prepared in this Example both had sufficient reverse transcription activity.

### Example 4: Thermal Stability Test of Reverse Transcriptases

Each of the reverse transcriptases G2 and G6 was diluted with a storage buffer (50 mM Tris-HCl (pH: 7.5), 300 mM KCl, 50% glycerol, 0.1 mM EDTA) to 10 U/µL and stored at 50°C or 55°C for 10 minutes. Thereafter, the reverse transcription activity was measured, and the residual activity of each variant reverse transcriptase after storage was determined. The residual activity can be calculated by dividing the reverse transcription activity value after storage by the reverse transcription activity value before storage, as shown in the following Formula I. Residual activity (%) = (reverse transcription activity value after storage/ reverse transcription activity value before storage) × 100

**Table 1**

| Name | Treatment conditions | Residual activity |
|---|---|---|
| WT | 50°C10min | 32% |
| | 55°C10min | 13% |
| G2 | 50°C10min | 80% |
| | 55°C10min | 29% |
| G6 | 50°C10min | 91% |
| | 55°C10min | 40% |

Table 1 shows that the residual activity of WT was 32% after heat treatment at 50°C for 10 minutes, while both the reverse transcriptases G2 and G6 showed a very high residual activity of about 80% to 90%. This indicates that each variant reverse transcriptase has significantly improved heat resistance. Even after heat treatment at a higher temperature of 55°C for 10 minutes, each variant reverse transcriptase showed a residual activity of about 30% or more, which was higher than that of wild-type reverse transcriptase. In particular, G6 showed a residual activity of 40% after heat treatment at 55°C for 10 minutes, indicating a significant improvement in thermal stability compared to WT. From the above, it was found that both the variant reverse transcriptases had improved thermal stability, and that the thermal stability of G6 was particularly significantly improved.

### Example 5: cDNA Synthesis Ability Test of Reverse Transcriptases

Each of the variant reverse transcriptases (G2 and G6) was diluted with a storage buffer (20 mM Tris-HCl (pH: 7.5), 100 mM NaCl, 50% glycerol, 0.1 mM EDTA, 1 mM DTT, 0.01% NP-40) to 10 U/µL. 1 µL of each diluted solution, 4 µL of reverse transcription reaction liquid 5×Buffer (produced by Toyobo Co., Ltd.), 2 µL of 10 mM dNTPs, 10 ng pf human total RNA, and 1 µL of 10 µM primer shown below were mixed with 11 µL of sterile water, and reverse transcription reactions were performed at 37°C, 42°C, 50°C, and 55°C for 20 minutes.
Reverse transcription primer: gttcgaccgtcttctcagcgctcc (SEQ ID NO: 7)

After the obtained cDNA was heated at 95°C for 5 minutes to inactivate the reverse transcriptase, 1 µL of the cDNA was subjected to PCR according to the instruction manual of KOD-Plus- (produced by Toyobo Co., Ltd.). 0.6 µL of 10 uM primer set was added. The sequences are shown below.
Forward primer: gccatgcatgtctgagtacgcacgg (SEQ ID NO: 8)
Reverse primer: tctagaattaccacagttatccaag (SEQ ID NO: 9)
The amplification product was subjected to electrophoresis using 1.5% agarose gel to confirm the presence or absence of amplification.

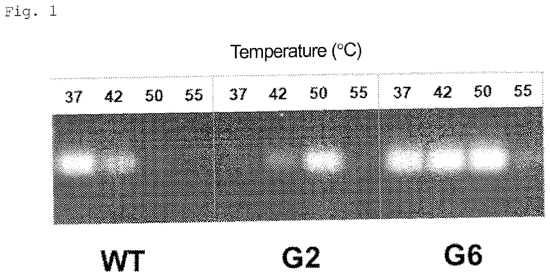

As shown in Fig. 1, in WT, no amplification is observed at a reverse transcription temperature of 50°C or higher. On the other hand, clear bands are observed for each variant reverse transcriptase even at a reverse transcription temperature of 50°C, indicating that the reverse transcription reaction sufficiently occurred. Further, in G6, amplification was observed even at a reverse transcription temperature of 55°C. From the above, it was found that each variant reverse transcriptase had significantly improved heat resistance compared to WT. In G2, there is almost no band at 37°C. This suggests that the optimal reaction temperature for the reverse transcriptase G2 may be around 50°C. On the other hand, the reverse transcriptase G6 was confirmed to exhibit reverse transcription activity in a wide temperature range of 37°C to 55°C.

### Example 6: Sequence Comparison between Variant Reverse Transcriptase and Wild-Type Reverse Transcriptase

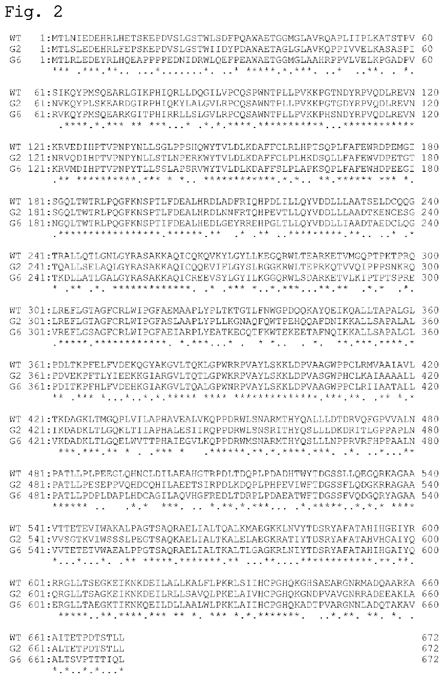

The amino acid sequence of each of the variant reverse transcriptases (G2 and G6), which were confirmed to have both reverse transcription activity and high thermal stability in the above Example, was compared with the amino acid sequence of wild-type reverse transcriptase to identify amino acid residues that characterize the variant reverse transcriptase. The results are shown in Fig. 2. As shown in Fig. 2, the variant reverse transcriptases have an amino acid sequence identity of 70% or less with the wild-type reverse transcriptase, indicating very low sequence similarity. Then, amino acid residues that were shared by the variant reverse transcriptases (G2 and G6), unlike the wild-type reverse transcriptase, were identified. As a result, 43 amino acid residues characterizing the variant reverse transcriptases (I5L, A47P, I51V, P52E, I62V, Q85L, I87V, V89R, P94A, H144R, R160P, I219T, T232D, S233T, L235E, Q266E, E276G, G338T, Q350K, T354S, Y377I, V414I, V419L, G425D, M429L, A463S, V476P, V477A, N495D, D519E, Y523F, E532D, T542V, Q569K, M573L, K578R, V581I, E597A, R600Q, S632A, E646V, R658K, and I662L; amino acid mutations from amino acid residues of the wild-type reverse transcriptase at positions corresponding to the amino acid sequences of SEQ ID NOs: 2 and 3) were found at positions corresponding to the loop regions of MMLV. It was also confirmed that many of these amino acid residues were present in the fingers and palm domains of MMLV and their vicinity. Among these amino acid residues, 21 amino acid residues (A47P, P52E, Q85L, V89R, P94A, R160P, I219T, T232D, L235E, Q266E, E276G, G338T, Q350K, Y377I, G425D, A463S, V476P, N495D, T542V, Q569K, E597A, R600Q, and E646V) were replaced with amino acid residues structurally and/or chemically different from the amino acid residues of the wild-type reverse transcriptase, which was assumed to contribute to the improvement of thermal stability.

### Example 7: Acquisition of Further Reverse Transcriptase and Sequence Comparison

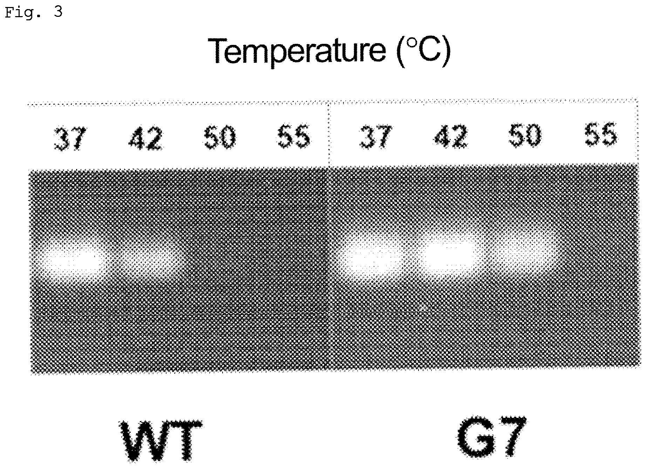

Another candidate amino acid sequence (G7, SEQ ID NO: 4) was further selected from the multiple candidate amino acid sequences obtained by screening in Example 1, and a reverse transcriptase was obtained in the same manner as in Examples 2 and 3. Specifically, a DNA sequence (SEQ ID NO: 10) was designed from the candidate amino acid sequence G7 based on the codon usage of Escherichia coli, and the resulting sequence was cloned into pET-23b(+) to produce a plasmid into which the candidate reverse transcriptase sequence was incorporated (pG7). The obtained plasmid was transformed into BL21-Gold Competent Cells (produced by Agilent Technologies). The cells were cultured under the same conditions as in Example 3, and a reverse transcriptase was obtained by purification from a cell disruption solution of the cells collected from the culture medium. The reverse transcription activity of the obtained reverse transcriptase (G7) after storage at 50°C for 10 minutes was measured in the same manner as in Example 4. The results are shown in Table 2 below. Further, reverse transcription reactions were performed at 37°C, 42°C, 50°C, and 55°C for 20 minutes in the same manner as in Example 5, and the presence or absence of amplification products after PCR was confirmed. The results are shown in Fig. 3.

**Table 2**

| Name | Treatment conditions | Residual activity |
|---|---|---|
| WT | 50°C10min | 32% |
| G7 | 50°C10min | 71% |

As shown in the results of Table 2 and Fig. 3, the reverse transcriptase G7 obtained in this Example was confirmed to have similar thermal stability to the reverse transcriptases G2 and G6. Fig. 4 shows the results of aligning the reverse transcriptase G7, reverse transcriptases G2 and G6, and wild-type reverse transcriptase. The amino acid sequence of the reverse transcriptase G7 was confirmed to have MDLAAA as six amino acid residues at positions 67, 175, 229, 308, 437, and 592 that characterized the reverse transcriptase. It was also confirmed that the amino acid sequence of the reverse transcriptase G7 commonly had, among the amino acid residues characteristic to the reverse transcriptases represented by SEQ ID NOs: 2 and 3, the following amino acids:
(2) P at a position corresponding to position 47,
(5) V at a position corresponding to position 62,
(10) R a position corresponding to position 144,
(14) T at a position corresponding to position 233,
(20) S at a position corresponding to position 354,
(25) L at a position corresponding to position 429,
(27) P at a position corresponding to position 476,
(29) D at a position corresponding to position 495,
(33) V at a position corresponding to position 542, and
(43) L at a position corresponding to position 662.
These amino acid residues were not found in the wild-type MMLV reverse transcriptase, but were commonly found in the reverse transcriptases G2, G6, and G7, which had similar thermal stability, suggesting that they were highly likely to contribute to the improvement of thermal stability.

On the other hand, it was confirmed that, of the amino acid sequences represented by SEQ ID NOs: 2 to 4, (A) a region corresponding to positions 91 to 105, (B) a region corresponding to positions 109 to 120, (C) a region corresponding to positions 125 to 138, (D) a region corresponding to positions 146 to 157, (E) a region corresponding to positions 182 to 205, (F) a region corresponding to positions 220 to 228, (G) a region corresponding to positions 251 to 263, (H) a region corresponding to positions 302 to 319, (I) a region corresponding to positions 351 to 358, and (J) a region corresponding to positions 391 to 404 (surrounded parts (A) to (J) in Fig. 4) showed high conservation with the respective corresponding regions of the amino acid sequence of the wild-type reverse transcriptase represented by SEQ ID NO: 1. From this result, it is considered that fewer mutations in these regions are desirable.

### Example 8: Acquisition of Reverse Transcriptases (G2-D583N and G6-D583N)

DNA sequences (SEQ ID NOs: 5 and 6) encoding the two reverse transcriptases (G2 and G6) produced in Example 2 were each cloned into pET23b(+) without tags to produce plasmids (pG2-2 and pG6-2) into which genes encoding each reverse transcriptase were incorporated. Using each of the obtained plasmids as a template, plasmids (pG2-2-D583N and pG6-2-D583N) into which mutation D583N was introduced were produced using the KOD-Plus-Mutagenesis Kit (produced by Toyobo Co., Ltd.). The method was according to the instruction manual, and the following primer sets were used for mutation introduction.
Forward primer (pG2-2): AACTCACGCTATGCGTTTGCGAC (SEQ ID NO: 11)
Reverse primer (pG2-2): CGTATAGATGGTTGCGCGTTTCC (SEQ ID NO: 12)
Forward primer (pG6-2): AACtgtgtaaatgttcagacgtttgcctgc (SEQ ID NO: 13)
Reverse primer (pG6-2): tgtgtaaatgttcagacgtttgcctgc (SEQ ID NO: 14)

The obtained two plasmids (pG2-2-D583N and pG6-2-D583N) were each transformed into BL21(DE3) Competent E. coli (produced by New England Biolabs) and used for enzyme preparation.

The two transformants obtained as described above were cultured as described below. 80 mL of sterilized TB medium (Molecular Cloning, 2nd Edition, p.A. 2) containing 100 µg/mL ampicillin was dispensed into a 500-mL Sakaguchi flask. In this medium, a transformant previously cultured at 37°C for 16 hours in 3 mL of LB medium (1% bactotrypton, 0.5% yeast extract, 0.5% sodium chloride) containing 100 ug/mL ampicillin was inoculated and aerobically cultured at 30°C for 16 hours. Then, IPTG (produced by Nacalai Tesque, Inc.) was added to a final concentration of 0.1 mM, and the cells were aerobically cultured at 30°C for another 4 hours. The cells were collected from the culture medium by centrifugation.

The obtained two types of cells were each purified as described below. 10 g of each cell was suspended in 20 mL of buffer 1 (20 mM tris-hydrochloric acid (pH: 7.5), 5 mM EDTA, 1 mM DTT, 100 mM NaCl). This was crushed using an ultrasonic crusher, and centrifuged at 12000 revolutions/min for 10 minutes to separate the precipitate. 0.4 mL of a 0.6% polyethyleneimine solution was added to the obtained supernatant, followed by stirring for 30 minutes. The resultant was centrifuged at 12000 revolutions/min for 10 minutes to separate the precipitate, and the supernatant was collected. 4.56 g of ammonium sulfate was added to this liquid, followed by stirring for 30 minutes. The resultant was centrifuged at 12000 revolutions/min for 10 minutes to separate and collect the precipitate.

The collected precipitate was dissolved in 5 mL of buffer 2 (20 mM tris-hydrochloric acid (pH: 7.5), 0.1 mM EDTA, 1 mM DTT, 50 mM NaCl, 10% glycerol), followed by desalting and buffer replacement with buffer 2 by dialysis. This was applied to a DEAE Sepharose column equilibrated with buffer 2 in advance, and the non-adsorbed fraction was collected. The obtained fraction was applied to a phosphocellulose column equilibrated with buffer 2 in advance, washed with buffer 2, and then eluted with a linear gradient of 50 to 500 mM NaCl using buffer 2 containing 500 mM NaCl.

Among the thus-obtained fractions, those having reverse transcriptase activity and not having RNase H activity were pooled. Next, the obtained fractions were subjected to desalting and buffer replacement with buffer 2 by dialysis. This was applied to a heparin-Sepharose column equilibrated with buffer 2 in advance, washed with buffer 2, and then eluted with a linear gradient of 50 to 1000 mM NaCl using buffer 2 containing 1000 mM NaCl, and fractions with reverse transcriptase activity were collected. Through the above operations, 10 mg of each reverse transcriptase showing a nearly single band on SDS-PAGE was obtained. The reverse transcriptase obtained from cells transformed with the plasmid pG2-2-D583N was named G2-D583N, and the reverse transcriptase obtained from cells transformed with the plasmid pG6-2-D583N was named G6-D583N. Each reverse transcriptase corresponds to a reverse transcriptase in which aspartic acid at position 583 of the amino acid sequences of G2 and G6 (position corresponding to position 584 of SEQ ID NO: 2 and SEQ ID NO: 3) is substituted with asparagine.

### Example 9: cDNA Synthesis Ability Test of Reverse Transcriptase (G2-D583N)

0.5 µL of an enzyme solution obtained by diluting the variant reverse transcriptase (G2-D583N) obtained in Example 8 with a storage buffer (20 mM Tris-HCl (pH: 7.5), 100 mM NaCl, 50% glycerol, 0.1 mM EDTA, 1 mM DTT, 0.01% NP-40) to 10 U/µL, 2 µL of 5×RT Buffer (included with ReverTra Ace (registered trademark) qPCR RT Kit (produced by Toyobo Co., Ltd.)), 0.5 µL of Primer mix (included with ReverTra Ace (registered trademark) qPCR RT Kit (produced by Toyobo Co., Ltd.)), and 500 to 0.005 ng of Total RNA-Human Tumor Cell Line: Hela (produced by Biochain) were mixed, and the final liquid volume was then adjusted to 10 µL. Reverse transcription reactions were performed at 37°C, 45°C, and 50°C for 15 minutes to synthesize cDNA.

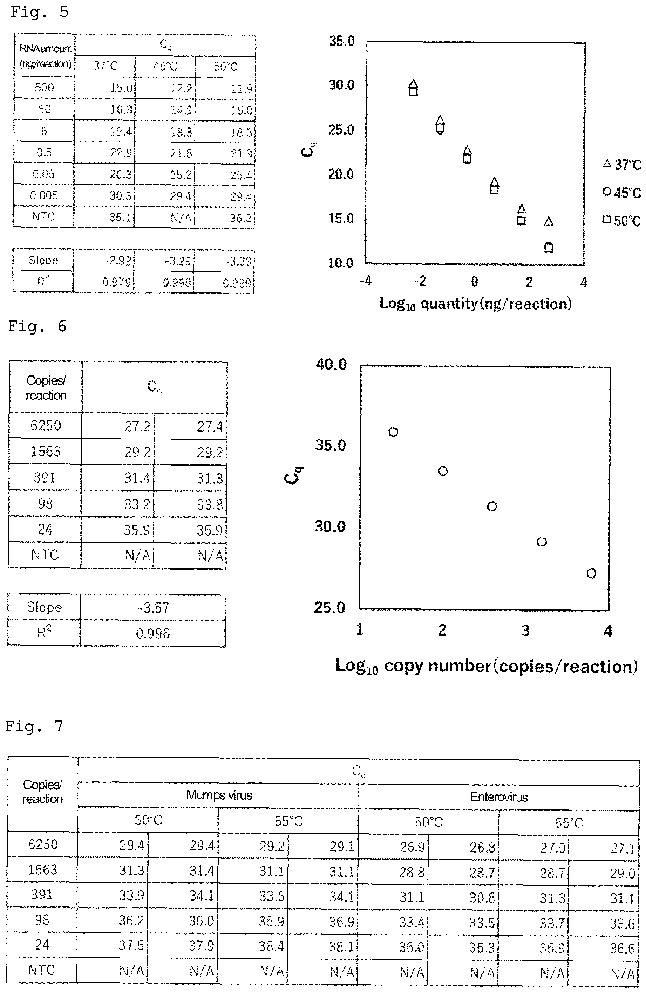

After the obtained cDNA was heated at 95°C for 5 minutes to inactivate the reverse transcriptase, real-time PCR was performed using 2 µL of the cDNA according to the instruction manual of THUNDERBIRD (registered trademark) SYBR (registered trademark) qPCR Mix (produced by Toyobo Co., Ltd.). The sequences of the primer set used are shown below. The measuring machine stand used was StepOnePlus produced by ThermoFisher Scientific. Fig. 5 shows a calibration curve obtained from the obtained Cq values and data.
Forward primer: AGAAAATCTGGCACCACACC (SEQ ID NO: 15)
Reverse primer: AGAGGCGTACAGGGATAGCA (SEQ ID NO: 16)

As shown in Fig. 5, when the reverse transcription reaction was performed at 37°C, the overall Cq values were large, and the linearity in regions with a large amount of RNA in the calibration curve was slightly disturbed, suggesting that the reverse transcription reaction did not progress sufficiently. On the other hand, under the conditions of 45°C and 50°C, the Cq values became small, and good linearity was observed even in regions with a large amount of RNA. This suggests that, as shown in Example 5, the optimal reaction temperature of the reverse transcriptase (G2-D583N) derived from G2 (SEQ ID NO: 2) may have shifted to a relatively high temperature range. Furthermore, the results of this Example showed that even if the amino acid sequence of SEQ ID NO: 2 contains a mutation, the effects of the present invention can be obtained.

### Example 10: One-Step qRT-PCR Using Reverse Transcriptase (G6-D583N)

0.5 µL of an enzyme solution obtained by diluting the variant reverse transcriptase (G6-D583N) obtained in Example 8 with a storage buffer (20 mM Tris-HCl (pH: 7.5), 100 mM NaCl, 50% glycerol, 0.1 mM EDTA, 1 mM DTT, 0.01% NP-40) to 6 U/µL, 10 µL of 2×Reaction Buffer (included with THUNDERBIRD (registered trademark) Probe One-step qRT-PCR Kit (produced by Toyobo Co., Ltd.)), 0.5 µL of DNA Polymerase (included with THUNDERBIRD (registered trademark) Probe One-step qRT-PCR Kit (produced by Toyobo Co., Ltd.)), 24 to 6250 copies of enterovirus RNA, 10 pmol of each of the primer set shown below, and 4 pmol of the probe shown below were mixed, and the final liquid volume was then adjusted to 20 µL. After a reverse transcription reaction was performed at 50°C for 10 minutes, one-step qRT-PCR was performed with a pre-reaction at 95°C, 60 seconds, followed by 45 cycles of 95°C, 15 seconds → 60°C, 45 seconds. The measuring machine stand used was StepOnePlus produced by ThermoFisher Scientific. Fig. 6 shows a calibration curve obtained from the obtained Cq values and data.
Forward primer: CCTCCGGCCCCTGAATG (SEQ ID NO: 17)
Reverse primer: ACCGGATGGCCAATCCAA (SEQ ID NO: 18)
Probe: CCGACTACTTTGGGTGTCCGTGTTTC (SEQ ID NO: 19)

As shown in Fig. 6, it was revealed that a wide range of copies of RNA could be detected by using the reverse transcriptase of the present invention (G6-D583N), and that the calibration curve showed good linearity. Therefore, it was confirmed that reverse transcription reactions can be performed even at high temperatures by using the reverse transcriptase (G6-D583N) derived from G6 (SEQ ID NO: 3), and that this reverse transcriptase can be suitably used in one-step qRT-PCR reactions. It was also shown that even when the amino acid sequence of SEQ ID NO: 3 contains a mutation, the effects of the present invention can be obtained.

### Example 11: One-Step qRT-PCR Using Reverse Transcriptase (G2-D583N)

0.5 µL of an enzyme solution obtained by diluting the variant reverse transcriptase (G2-D583N) obtained in Example 8 with a storage buffer (20 mM Tris-HCl (pH: 7.5), 100 mM NaCl, 50% glycerol, 0.1 mM EDTA, 1 mM DTT, 0.01% NP-40) to 6 U/µL, 10 µL of 2×Reaction Buffer (included with THUNDERBIRD (registered trademark) Probe One-step qRT-PCR Kit (produced by Toyobo Co., Ltd.)), 0.5 µL of DNA Polymerase (included with THUNDERBIRD (registered trademark) Probe One-step qRT-PCR Kit (produced by Toyobo Co., Ltd.)), 24 to 6250 copies of enterovirus RNA, 10 pmol of each of the primer set described in Example 10, and 4 pmol of the probe described in Example 10 were mixed, and the final liquid volume was then adjusted to 20 µL. After a reverse transcription reaction was performed at 50°C or 55°C for 10 minutes, one-step qRT-PCR was performed with a pre-reaction at 95°C, 60 seconds, followed by 45 cycles of 95°C, 15 seconds → 60°C, 45 seconds. The measuring machine stand used was StepOnePlus produced by ThermoFisher Scientific.

Further, one-step qRT-PCR was also performed in the same manner as described above using mumps virus RNA in place of the enterovirus. The primer set and probe used therein were changed as shown below. The obtained Cq values are shown in Fig. 7.
Forward primer: GTGACCCTGCCGTTGCA (SEQ ID NO: 20)
Reverse primer: GTTATGATCAGAGAGAGAAGAATTAGCAATAG (SEQ ID NO: 21)
Probe: TATGCCGGCGATCCAACCTCCCTTATA (SEQ ID NO: 22)

As shown in Fig. 7, it was confirmed that by using the reverse transcriptase of the present invention (G2-D583N), a wide range of copies of RNA could be detected by reverse transcription reactions at high temperatures, and that the reverse transcriptase of the present invention can also be suitably used in one-step qRT-PCR reactions.

### Industrial Applicability

The present invention provides a novel reverse transcriptase with excellent thermal stability useful in the field of molecular biology, and a reagent, kit, and the like containing the reverse transcriptase. The present invention is particularly useful for gene expression analysis, and is highly versatile and highly convenient; therefore, the present invention can be used not only for research but also for clinical diagnosis, environmental tests, and the like.

## Claims

1. A reverse transcriptase comprising the following amino acid sequence:
(a) an amino acid sequence having at least 75% identity with the amino acid sequence represented by SEQ ID NO: 2 or 3; or
(b) an amino acid sequence having deletion, substitution, insertion, or addition of one or several amino acids in the amino acid sequence represented by SEQ ID NO: 2 or 3.

2. The reverse transcriptase according to claim 1, which has the following amino acid residues (i) to (vi) in the amino acid sequence (a) or (b):
(i) an amino acid at a position corresponding to position 67 is L or M,
(ii) an amino acid at a position corresponding to position 175 is D,
(iii) an amino acid at a position corresponding to position 229 is L or I,
(iv) an amino acid at a position corresponding to position 308 is A,
(v) an amino acid at a position corresponding to position 437 is A or T, and
(vi) an amino acid at a position corresponding to position 592 is A.

3. The reverse transcriptase according to claim 1 or 2, which has at least one amino acid residue selected from the group consisting of the following (1) to (43) in the amino acid sequence (a) or (b):
(1) an amino acid at a position corresponding to position 5 is L,
(2) an amino acid at a position corresponding to position 47 is P,
(3) an amino acid at a position corresponding to position 51 is V,
(4) an amino acid at a position corresponding to position 52 is E,
(5) an amino acid at a position corresponding to position 62 is V,
(6) an amino acid at a position corresponding to position 85 is L,
(7) an amino acid at a position corresponding to position 87 is V,
(8) an amino acid at a position corresponding to position 89 is R,
(9) an amino acid at a position corresponding to position 94 is A,
(10) an amino acid at a position corresponding to position 144 is R,
(11) an amino acid at a position corresponding to position 160 is P,
(12) an amino acid at a position corresponding to position 219 is T,
(13) an amino acid at a position corresponding to position 232 is D,
(14) an amino acid at a position corresponding to position 233 is T,
(15) an amino acid at a position corresponding to position 235 is E,
(16) an amino acid at a position corresponding to position 266 is E,
(17) an amino acid at a position corresponding to position 276 is G,
(18) an amino acid at a position corresponding to position 338 is T,
(19) an amino acid at a position corresponding to position 350 is K,
(20) an amino acid at a position corresponding to position 354 is S,
(21) an amino acid at a position corresponding to position 377 is I,
(22) an amino acid at a position corresponding to position 414 is I,
(23) an amino acid at a position corresponding to position 419 is L,
(24) an amino acid at a position corresponding to position 425 is D,
(25) an amino acid at a position corresponding to position 429 is L,
(26) an amino acid at a position corresponding to position 463 is S,
(27) an amino acid at a position corresponding to position 476 is P,
(28) an amino acid at a position corresponding to position 477 is A,
(29) an amino acid at a position corresponding to position 495 is D,
(30) an amino acid at a position corresponding to position 519 is E,
(31) an amino acid at a position corresponding to position 523 is F,
(32) an amino acid at a position corresponding to position 532 is D,
(33) an amino acid at a position corresponding to position 542 is V,
(34) an amino acid at a position corresponding to position 569 is K,
(35) an amino acid at a position corresponding to position 573 is L,
(36) an amino acid at a position corresponding to position 578 is R,
(37) an amino acid at a position corresponding to position 581 is I,
(38) an amino acid at a position corresponding to position 597 is A,
(39) an amino acid at a position corresponding to position 600 is Q,
(40) an amino acid at a position corresponding to position 632 is A,
(41) an amino acid at a position corresponding to position 646 is V,
(42) an amino acid at a position corresponding to position 658 is K, and
(43) an amino acid at a position corresponding to position 662 is L.

4. The reverse transcriptase according to any one of claims 1 to 3, which has at least one amino acid residue selected from the group consisting of the following (2), (4), (6), (8), (9), (11), (12), (13), (15), (16), (19), (21), (24), (26), (27), (29), (33), (34), (38), (39), and (41) in the amino acid sequence (a) or (b) :
(2) an amino acid at a position corresponding to position 47 is P,
(4) an amino acid at a position corresponding to position 52 is E,
(6) an amino acid at a position corresponding to position 85 is L,
(8) an amino acid at a position corresponding to position 89 is R,
(9) an amino acid at a position corresponding to position 94 is A,
(11) an amino acid at a position corresponding to position 160 is P,
(12) an amino acid at a position corresponding to position 219 is T,
(13) an amino acid at a position corresponding to position 232 is D,
(15) an amino acid at a position corresponding to position 235 is E,
(16) an amino acid at a position corresponding to position 266 is E,
(19) an amino acid at a position corresponding to position 350 is K,
(21) an amino acid at a position corresponding to position 377 is I,
(24) an amino acid at a position corresponding to position 425 is D,
(26) an amino acid at a position corresponding to position 463 is S,
(27) an amino acid at a position corresponding to position 476 is P,
(29) an amino acid at a position corresponding to position 495 is D,
(33) an amino acid at a position corresponding to position 542 is V,
(34) an amino acid at a position corresponding to position 569 is K,
(38) an amino acid at a position corresponding to position 597 is A,
(39) an amino acid at a position corresponding to position 600 is Q, and
(41) an amino acid at a position corresponding to position 646 is V.

5. The reverse transcriptase according to any one of claims 1 to 4, which has at least one amino acid residue selected from the group consisting of the following (2), (5), (10), (14), (20), (25), (27), (29), (33), and (43) in the amino acid sequence (a) or (b) :
(2) an amino acid at a position corresponding to position 47 is P,
(5) an amino acid at a position corresponding to position 62 is V,
(10) an amino acid at a position corresponding to position 144 is R,
(14) an amino acid at a position corresponding to position 233 is T,
(20) an amino acid at a position corresponding to position 354 is S,
(25) an amino acid at a position corresponding to position 429 is L,
(27) an amino acid at a position corresponding to position 476 is P,
(29) an amino acid at a position corresponding to position 495 is D,
(33) an amino acid at a position corresponding to position 542 is V, and
(43) an amino acid at a position corresponding to position 662 is L.

6. A reverse transcriptase having at least 80% identity with the amino acid sequence represented by SEQ ID NO: 1, and having at least one amino acid residue selected from the group consisting of the following (1) to (43) :
(1) an amino acid at a position corresponding to position 5 is L,
(2) an amino acid at a position corresponding to position 47 is P,
(3) an amino acid at a position corresponding to position 51 is V,
(4) an amino acid at a position corresponding to position 52 is E,
(5) an amino acid at a position corresponding to position 62 is V,
(6) an amino acid at a position corresponding to position 85 is L,
(7) an amino acid at a position corresponding to position 87 is V,
(8) an amino acid at a position corresponding to position 89 is R,
(9) an amino acid at a position corresponding to position 94 is A,
(10) an amino acid at a position corresponding to position 144 is R,
(11) an amino acid at a position corresponding to position 160 is P,
(12) an amino acid at a position corresponding to position 219 is T,
(13) an amino acid at a position corresponding to position 232 is D,
(14) an amino acid at a position corresponding to position 233 is T,
(15) an amino acid at a position corresponding to position 235 is E,
(16) an amino acid at a position corresponding to position 266 is E,
(17) an amino acid at a position corresponding to position 276 is G,
(18) an amino acid at a position corresponding to position 338 is T,
(19) an amino acid at a position corresponding to position 350 is K,
(20) an amino acid at a position corresponding to position 354 is S,
(21) an amino acid at a position corresponding to position 377 is I,
(22) an amino acid at a position corresponding to position 414 is I,
(23) an amino acid at a position corresponding to position 419 is L,
(24) an amino acid at a position corresponding to position 425 is D,
(25) an amino acid at a position corresponding to position 429 is L,
(26) an amino acid at a position corresponding to position 463 is S,
(27) an amino acid at a position corresponding to position 476 is P,
(28) an amino acid at a position corresponding to position 477 is A,
(29) an amino acid at a position corresponding to position 495 is D,
(30) an amino acid at a position corresponding to position 519 is E,
(31) an amino acid at a position corresponding to position 523 is F,
(32) an amino acid at a position corresponding to position 532 is D,
(33) an amino acid at a position corresponding to position 542 is V,
(34) an amino acid at a position corresponding to position 569 is K,
(35) an amino acid at a position corresponding to position 573 is L,
(36) an amino acid at a position corresponding to position 578 is R,
(37) an amino acid at a position corresponding to position 581 is I,
(38) an amino acid at a position corresponding to position 597 is A,
(39) an amino acid at a position corresponding to position 600 is Q,
(40) an amino acid at a position corresponding to position 632 is A,
(41) an amino acid at a position corresponding to position 646 is V,
(42) an amino acid at a position corresponding to position 658 is K, and
(43) an amino acid at a position corresponding to position 662 is L.

7. The reverse transcriptase according to claim 6, which has at least 80% identity with the amino acid sequence represented by SEQ ID NO: 1, and has at least one amino acid residue selected from the group consisting of the following (2), (4), (6), (8), (9), (11), (12), (13), (15), (16), (19), (21), (24), (26), (27), (29), (33), (34), (38), (39), and (41):
(2) an amino acid at a position corresponding to position 47 is P,
(4) an amino acid at a position corresponding to position 52 is E,
(6) an amino acid at a position corresponding to position 85 is L,
(8) an amino acid at a position corresponding to position 89 is R,
(9) an amino acid at a position corresponding to position 94 is A,
(11) an amino acid at a position corresponding to position 160 is P,
(12) an amino acid at a position corresponding to position 219 is T,
(13) an amino acid at a position corresponding to position 232 is D,
(15) an amino acid at a position corresponding to position 235 is E,
(16) an amino acid at a position corresponding to position 266 is E,
(19) an amino acid at a position corresponding to position 350 is K,
(21) an amino acid at a position corresponding to position 377 is I,
(24) an amino acid at a position corresponding to position 425 is D,
(26) an amino acid at a position corresponding to position 463 is S,
(27) an amino acid at a position corresponding to position 476 is P,
(29) an amino acid at a position corresponding to position 495 is D,
(33) an amino acid at a position corresponding to position 542 is V,
(34) an amino acid at a position corresponding to position 569 is K,
(38) an amino acid at a position corresponding to position 597 is A,
(39) an amino acid at a position corresponding to position 600 is Q, and
(41) an amino acid at a position corresponding to position 646 is V.

8. The reverse transcriptase according to claim 6 or 7, which has at least 80% identity with the amino acid sequence represented by SEQ ID NO: 1, and has at least one amino acid residue selected from the group consisting of the following (2), (5), (10), (14), (20), (25), (27), (29), (33), and (43):
(2) an amino acid at a position corresponding to position 47 is P,
(5) an amino acid at a position corresponding to position 62 is V,
(10) an amino acid at a position corresponding to position 144 is R,
(14) an amino acid at a position corresponding to position 233 is T,
(20) an amino acid at a position corresponding to position 354 is S,
(25) an amino acid at a position corresponding to position 429 is L,
(27) an amino acid at a position corresponding to position 476 is P,
(29) an amino acid at a position corresponding to position 495 is D,
(33) an amino acid at a position corresponding to position 542 is V, and
(43) an amino acid at a position corresponding to position 662 is L.

9. The reverse transcriptase according to any one of claims 1 to 8, wherein an amino acid sequence of one or more regions selected from the group consisting of (A) a region corresponding to positions 91 to 105, (B) a region corresponding to positions 109 to 120, (C) a region corresponding to positions 125 to 138, (D) a region corresponding to positions 146 to 157, (E) a region corresponding to positions 182 to 205, (F) a region corresponding to positions 220 to 228, (G) a region corresponding to positions 251 to 263, (H) a region corresponding to positions 302 to 319, (I) a region corresponding to positions 351 to 358, and (J) a region corresponding to positions 391 to 404 of the amino acid sequence represented by SEQ ID NO: 1, 2, or 3 has at least 90% identity with an amino acid sequence of a corresponding region in the amino acid sequence of SEQ ID NO: 1, 2, or 3.

10. The reverse transcriptase according to any one of claims 1 to 9, wherein the amino acid sequence (a) is an amino acid sequence having at least 85% identity with the amino acid sequence represented by SEQ ID NO: 2 or 3.

11. The reverse transcriptase according to any one of claims 1 to 10, which lacks RNase H activity.

12. The reverse transcriptase according to any one of claims 1 to 11, which has a residual activity of 70% or more when heated at 50°C for 10 minutes.

13. The reverse transcriptase according to any one of claims 1 to 12, which has a residual activity of 20% or more when heated at 55°C for 10 minutes.

14. A polynucleotide encoding the reverse transcriptase according to any one of claims 1 to 13.

15. A vector comprising the polynucleotide according to claim 14.

16. A cell transformed with the vector according to claim 15.

17. A reagent comprising the reverse transcriptase according to any one of claims 1 to 13, the polynucleotide according to claim 14, the vector according to claim 15, and/or the cell according to claim 16.

18. A method for producing the reverse transcriptase according to any one of claims 1 to 13 using the polynucleotide according to claim 14, the vector according to claim 15, the cell according to claim 16, and/or the reagent according to claim 17.

19. A method for synthesizing cDNA from an RNA template using the reverse transcriptase according to any one of claims 1 to 13.

20. The method according to claim 19, which is a RT-PCR method further comprising performing a PCR reaction.

21. A kit comprising the reverse transcriptase according to any one of claims 1 to 13.

22. The kit according to claim 21, for use in synthesis of cDNA using RNA as a template.
